# EUROPEAN PATENT SPECIFICATION

(11) **EP 3 097 921 B1**
(45) Date of publication and mention of the grant of the patent: **23.06.2021**
(21) Application number: 16171535.4
(22) Date of filing: 26.05.2016
(51) Int. Cl.: A61K 36/9066, A61P 1/00, A61K 31/045

(54) **COMPOSITIONS COMPRISING GERANIOL AND DRY GINGER FOR USE IN THE TREATMENT OF IRRITABLE BOWEL SYNDROME**
ZUSAMMENSETZUNGEN AUS GERANIOL UND TROCKENINGWER ZUR BEHANDLUNG VON REIZBAREM BOWEL-SYNDROM
COMPOSITIONS COMPRENANT DU GÉRANIOL ET DU GINGEMBRE SEC À UTILISER DANS LE TRAITEMENT DU SYNDROME DE L'INTESTIN IRRITABLE

(30) Priority: 27.05.2015 IT UB20151212
(43) Date of publication of application: 30.11.2016
(73) Proprietor: Targeting Gut Disease S.r.l., 40127 Bologna (IT)
(72) Inventor: VALERII, Maria Chiara, 40131 BOLOGNA (IT)
(74) Representative: Longoni, Alessandra

(56) References cited:
- WO-A1-2012/013495
- WO-A2-2005/107728
- FR-A1- 2 572 935
- FR-A1- 2 762 994
- JP-A- 2013 126 961
- US-A1- 2006 228 397
- US-A1- 2006 280 835
- US-A1- 2008 027 024
- US-A1- 2008 145 409
- US-A1- 2009 175 982
- US-B1- 6 210 686
- JAMES W ANDERSON ET AL: "Health benefits of dietary fiber", NUTRITION REVIEWS., vol. 67, no. 4, 1 April 2009 (2009-04-01), pages 188-205, XP055234434, US ISSN: 0029-6643, DOI: 10.1111/j.1753-4887.2009.00189.x
- KATHARINNE INGRID MORAES DE CARVALHO ET AL: "Geraniol-a flavoring agent with multifunctional effects in protecting the gastric and duodenal mucosa", NAUNYN-SCHMIEDEBERG'S ARCHIVES OF PHARMACOLOGY, vol. 387, no. 4, 1 April 2014 (2014-04-01) , pages 355-365, XP055233855, DE ISSN: 0028-1298, DOI: 10.1007/s00210-013-0947-z
- KATAOKA G ET AL: "Helicobacter pylori motility inhibitor used for preventing and treating Helicobacter pylori infection, and used in food and drinks, contains monoterpene compound, particularly beta myrcene", WPI / THOMSON,, vol. 2012, no. 30, 19 April 2012 (2012-04-19), XP002751949,
- Sushil K Garg ET AL: "Curcumin for maintenance of remission in ulcerative colitis" In: "Cochrane Database of Systematic Reviews", 17 October 2012 (2012-10-17), John Wiley & Sons, Ltd, Chichester, UK, XP055234140, DOI: 10.1002/14651858.CD008424.pub2, * the whole document *
- ROJA RAHIMI ET AL: "On the Use of Herbal Medicines in Management of Inflammatory Bowel Diseases: A Systematic Review of Animal and Human Studies", DIGESTIVE DISEASES AND SCIENCES, KLUWER ACADEMIC PUBLISHERS-PLENUM PUBLISHERS, NE, vol. 54, no. 3, 10 July 2008 (2008-07-10), pages 471-480, XP019673418, ISSN: 1573-2568
- SHEREHAN M. IBRAHIM ET AL: "Geraniol, Alone and in Combination with Pioglitazone, Ameliorates Fructose-Induced Metabolic Syndrome in Rats via the Modulation of Both Inflammatory and Oxidative Stress Status", PLOS ONE, vol. 10, no. 2, 1 January 2015 (2015-01-01), page e0117516, XP055233852, US ISSN: 1932-6203, DOI: 10.1371/journal.pone.0117516
- JOSHUA K KO ET AL: "Ginger extract and polaprezinc exert gastroprotective actions by anti-oxidant and growth factor modulating effects in rats", JOURNAL OF GASTROENTEROLOGY AND HEPATOLOGY, vol. 25, no. 12, 23 December 2010 (2010-12-23), pages 1861-1869, XP055234418, AU ISSN: 0815-9319, DOI: 10.1111/j.1440-1746.2010.06347.x
- TUNG-TING SHAM ET AL: "A Review of the Phytochemistry and Pharmacological Activities of Raphani Semen", EVIDENCE-BASED COMPLEMENTARY AND ALTERNATIVE MEDICINE : ECAM, vol. 2, no. 4, 1 January 2013 (2013-01-01) , pages 337-16, XP055289668, United States ISSN: 1741-427X, DOI: 10.3390/toxins2092289
- None

## Description

This invention is defined in claim 1 and relates to a composition, or a mixture, comprising active ingredients of vegetable origin, in particular a mixture of active ingredients of vegetable origin or synthetic equivalents or extracts of vegetable origin which cotains essential oils or their components. Herein described is also a human or veterinary therapeutic use of a composition.

The essential oils and/or their active components have biological activity which is particularly attractive in particular in the case of oral consumption.

Conveniently dosed, they can be used for their germicidal, fungicidal, antibacterial, anti-inflammatory, anti-parasite and antibiotic activities (Thapa et al., 2012, Microbiology, 158 (11); 2870-2877). However, these substances present enormous problems in therapeutic applications due both to their aggressiveness with regard to the oral, oesophagus and gastric mucus and their toxic nature.

Even using correct therapeutic doses, extract of a vegetable origin which contain essential oils and/or their components (included synthethic equivalents) may cause inflammations, irritations, erosions or other damage to the mucus with they come into contact, such as, for examle, in the mouth.

Moreover, if, for example, a therapeutic action of essential oils is sought in the large intestine, it is very difficult to carry the corresponding active ingredients to the stretches of interest without damaging, even in a significant manner, the organs passed through before reaching the area in question.

In this context, the main aim of this invention is to obviate the above-mentioned drawbacks.

The aim of this invention is to provide a composition comprising active ingredients of a vegetable origin wherein their negative effects on the mucus are annulled or significantly reduced.

A further aim of this invention is to propose a therapeutic use of a composition according to the invention.

The technical purpose and aims specified are substantially achieved by a composition for use according to claim 1.

Further features and advantages of the invention are more apparent in the non-limiting description which follows of a preferred non-exclusive embodiment of a composition comprising active ingredients of vegetable origin.

Herein described is a composition which comprises at least a component of an essential oil, or a synthetic equivalent of the component, comprising at least an active ingredient.

Advantageously, the composition comprises a mixture of active ingredients. The composition herein described comprises at least an active ingredient, consisting preferably of an essential oil or its components or synthetic equivalents of the components.

The composition comprises an essential oil, preferably comprising the above-mentioned component of essential oil, and the corresponding active ingredient.

In practice, use is made of an oil essential for preparing at least one active ingredient of it.

The above-mentioned components of an essential oil or the essential oil are obtained as extracts of vegetable origin.

Examples of vegetable extract, herein described, are essential oils such as cinnamon oil, clove oil, thyme oil, rosemary oil, sage oil, bergamot oil.

Examples of active ingredients as components of the essential oils are eugenol, cinnamaldehyde, isoeugenol, thymol, carvacrol.

The term "synthetic equivalent" is used to indicate active ingredients obtained by synthesis or hemi-synthesis the structure of which is similar to or at least partly imitates the structure of the active principles of a vegetable origin mentioned above.

Synthetic equivalents of the active ingredients are, for example, synthetic equivalents of eugenol and cinnamaldehyde.

The active ingredient is present in the composition in quantities comprised between 5% and 50% in weight, preferably in quantities comprised between 10% and 25% in weight.

The active ingredient or the mixture of active ingredients is present in the composition in a quantity of between 10% and 50% in weight,

The composition comprises, mixed with the component of essential oil, or with the active ingredient, at least a dry plant extract or root or sprout or fruit extract comprising a quantity of soluble or insoluble fibre equal to or greater than 9% in dry weight.

Advantageously, the dry extract allows the active ingredient, adsorbed in the above-mentioned fibre, to be absorbed and metabolised in the large intestine.

Alternatively or in combination, the composition comprises, mixed with the component of oil essential, a soluble or insoluble synthetic vegetable fibre in a quantity corresponding to the quantity contained in a corresponding dry extract.

Examples of dry extract are dry extracts of glucomannan, turmeric root, boswellia, radish and fabaceae seeds.

Preferably, the dry extract is present in the composition in quantities comprised between 50% and 95% in weight, preferably in quantities comprised between 75% and 90% in weight.

Preferably, the dry extract has a grain size comprised in a range of from -1 to +8 of the Krumbein (φ) phi scale (1934).

In practice, in this composition, the dry extract defines a carrier which is rich in soluble and/or insoluble fibre.

Herein described is a process for preparing a composition as described above.

The process comprises a step of preparing a predetermined quantity, for example within the above-mentioned values, of the active ingredient, preferably in the form of a component of an essential oil.

A predetermined quantity of the dry extract in continuous agitation is added to the active ingredient, for example within the values described above.

After the addition of the dry extract in continuous agitation solid spherical lumps of variable dimensions are formed.

The process comprises a step of breaking the lumps by mechanical action and, preferably, a step of mixing the active ingredient and the dry extract until complete dissolution of the lumps and absorption of the active ingredient.

Advantageously, a composition according to the invention can be encapsulated in normal gelatine capsules, designed to contain liquid, solid and/or powder substances.

In this way, the compositions of the invention allow the encapsulation of the active ingredient, which is normally volatile, in normal gelatine capsules, used to contain liquid, solid and /or powder substances.

Herein described is a human or veterinary therapeutic use of a composition as described above comprising one or more active ingredients (essential oils or their components, extracts of vegetable origin which they contain, including synthetic equivalents) and one or more dry extracts of roots of plant, root, sprout or fruit, which is rich in fibre or soluble or insoluble synthetic vegetable fibre.

In one embodiment, a medication for therapeutic use comprises the composition as described and, preferably, at least one pharmaceutically acceptable carrier (such as capsules and drops, with immediate or delayed release).

In one embodiment, the composition is administered orally or rectally using carriers substantially known for the formulation of suspensions, included glycerol, cocoa-butter, beeswax and others.

In one embodiment, the composition comprises sweeteners and/or aromas and/or excipients for the encapsulation or for the formulation in tablets or granulated mixtures or suspensions, or natural or synthetic excipients synthesis to improve the transfer.

A dose for human or veterinary use herein described is between 0.5 mg and 10 grams/day of composition, for example on the basis of the patient, therapeutic requirements, means of administration, weight and age of the subject.

In a preferred embodiment of the composition for the treatment of Irritable Bowel Syndrome (IBS), the component of essential oil, that is, the active ingredient, comprises geraniol and the dry extract comprises dry extract of ginger root.

Herein described is a composition for the treatment of Irritable Bowel Syndrome, the component of essential oil, that is, the active ingredient, comprises geraniol and the dry extract comprises dry extract of turmeric root. Herein described is a composition for the treatment of Irritable Bowel Syndrome, the component of essential oil, that is, the active ingredient, comprises geraniol and the dry extract comprises dry extract of boswellia.

Herein described is a composition for the treatment of obesity, the component of essential oil, that is, the active ingredient, comprises geraniol and the dry extract comprises dry extract of fabaceae seeds or radish sprouts or black cabbage.

### Example 1.

A composition, comprising:
- natural or synthetic non-oxidised geraniol;
- dry extract of ginger root in powder.

45 g of dry extract of ginger root in powder are added to 20 g of pure natural or synthetic geraniol.

After the addition of the dry extract of ginger root in powder to the pure natural or synthetic geraniol, in continuous agitation, solid spherical lumps of variable dimensions are formed.

The lumps are broken by mechanical action (using, for example, a vibromixer or similar instrument) and the mixture is mixed until complete dissolution of the aggregates and complete absorption of the active ingredient.

### Example 2. (not part of the invention)

A composition, comprising:
- natural or synthetic non-oxidised geraniol;
- dry extract of turmeric root in powder.

35 g of dry extract of turmeric root in powder are added to 10 g of pure natural or synthetic geraniol.

After the addition of the dry extract of turmeric in powder to the natural or synthetic geraniol, in continuous agitation, solid spherical lumps of variable dimensions are formed.

The lumps are broken by mechanical action (using, for example, a vibromixer or similar instrument designed to break the aggregates) and the mixture is thus mixed until complete dissolution of the aggregates and complete absorption of the active ingredient.

### Example 3. (not part of the invention)

A composition, comprising:
- natural or synthetic geraniol;
- dry extract of sprouts in powder.

30 g of dry extract of sprouts in powder are added to 10 g of natural geraniol. After the addition of the dry extract of in powder to the natural or synthetic geraniol, in continuous agitation, solid spherical lumps of variable dimensions are formed.

The lumps are broken by mechanical action (using, for example, a vibromixer or similar instrument designed to break the aggregates) and the mixture is thus mixed until complete dissolution of the aggregates and complete absorption of the active ingredient.

### Example 4. (not part of the invention)

A composition, comprising:
- natural or synthetic non-oxidised geraniol and cinnamaldehyde;
- dry extract of radish sprouts in powder.

30 g of dry extract of radish sprouts in powder added to 5 g of natural non-oxidised geraniol, pre-mixed with 5 g of natural, non-oxidised cinnamaldehyde.

After the addition of the dry extract of radish sprouts in powder to the mixture of natural or synthetic geraniol and cinnamaldehyde, in continuous agitation, solid spherical lumps of variable dimensions are formed.

The lumps are broken by mechanical action (using, for example, a vibromixer or similar instrument designed to break the aggregates) and the mixture is thus mixed until complete dissolution of the aggregates and complete absorption of the active ingredient.

With reference to the above-mentioned examples, some results are provided below of animal and/or clinical trials supporting the therapeutic use.

Demonstration of the anti-colitic, anti-dysbiotic and anti-inflammatory effect of a composition according to example 1 in the treatment of patients suffering from irritable bowel syndrome.

The composition of example 1 has been administered to two human patients suffering from irritable bowel syndrome, with a dose of 800 mg/day (taken during main meals) for a cycle with a duration of 4 weeks. The symptomatology typical of irritable bowel syndrome (irregular alvus, abdominal swelling, urgent defecation) disappeared after treatment. The effectiveness of the treatment has been characterised by blood and faecal analyses.

Demonstration of the anti-colitic, anti-dysbiotic and anti-inflammatory effect of a composition according to example 1 in the treatment of patients suffering from bowel inflammation.

The composition of example 1 has been administered to two human patients suffering from bowel inflammation, with a dose of 800 mg/day (taken during main meals) for a cycle with a duration of 4 weeks. The symptomatology typical of colitis (diarrhoea, irregular alvus, abdominal cramps, urgent defecation) disappeared after treatment. The effectiveness of the treatment has been characterised by blood and faecal analyses.

Demonstration of the anti-colitic, anti-dysbiotic and anti-inflammatory effect of a composition according to example 2 in the treatment of patients suffering from irritable bowel syndrome.

The composition of example 2 has been administered to a human patient suffering from indeterminate colitis, with a dose of 800 mg/day (taken during main meals) for a cycle with a duration of 4 weeks. The symptomatology typical of colitis (irregular alvus, abdominal pains) reduced significantly after a week's treatment. The effectiveness of the treatment has been characterised by blood and faecal analyses. Demonstration of the weight loss effect of a composition according to example 3 in the treatment of adult mice of the C57BL/6 strain.

The composition of example 3 has been administered to a group of mice, with a daily dose of dose of 120 mg/kg for a cycle with a duration of 4 weeks. The effectiveness of the treatment was demonstrated by monitoring the weight of the animals, which were well fed with a weight-gaining diet with a high lipid content. After 4 weeks of treatment the group of animals treated had an average weight reduced by approximately 8%-10% compared with the group of control animals. The animals treated also showed a healthier intestinal microbiota compared with the control mice. This invention therefore relates to a new mixing of active agents mixed with natural or synthetic vegetable fibre and used for therapy on humans as claimed.

The composition is a damp powder, in particular oily, which releases the active component, in particular the active ingredient, in the zone of interest, that is to say, where the fibre is degraded, in particular in the large intestine.

Basically, the dry extracts, rich in fibre, harness the active component and release it when the fibre is degraded.

The fibre prevents the evaporation of the essential oil component, that is to say, the active ingredient, and therefore allows the encapsulation in wrappers of any type.

The compositions according to this invention are particularly advantageous since the mixture obtained is stable and the active component of the mixture (essential oils, their components or extracts of a vegetable origin which they contain, including synthetic equivalents), is adsorbed on a matrix which eliminates the majority of, or completely, the negative effects on the mucus, allowing a slow and controlled release, in particular along the final intestinal tract.

From a biological point of view, the mixture according to the invention allows the active ingredient to be taken free of the side effects which there would be without mixing. It also allows a slower release of the active ingredient (retard effect), which, whilst starting in the stomach, occurs mainly in the large intestine where the fibre is demolished by the intestinal microbiota (bacterial flora) and the active ingredient may perform its germicidal, fungicidal, antibacterial, anti-inflammatory, anti-parasite and antibiotic actions.

## Claims

1. A composition comprising at least a component of an essential oil comprising at least geraniol as active ingredient, the composition comprising at least a dry extract of ginger root equal to or greater than 9% in dry weight, the component being adsorbed in the dry extract which defines a carrier for the component, the composition resulting in an oily powder which releases the active ingredient in the large intestine for use in the treatment of irritable bowel syndrome (IBS), preferably at a therapeutic dose of geraniol equal to or greater than 50 mg/day.

## Patentansprüche

1. Zusammensetzung, umfassend mindestens eine Komponente eines ätherischen Öls, umfassend mindestens Geraniol als Wirkstoff, die Zusammensetzung umfassend mindestens einen Trockenextrakt aus Ingwerwurzel, der gleich wie oder größer als 9 % des Trockengewichts ist, wobei die Komponente in dem Trockenextrakt adsorbiert ist, der einen Träger für die Komponente definiert, wobei die Zusammensetzung in einem öligen Pulver resultiert, das den Wirkstoff in dem Dickdarm freisetzt, zur Verwendung bei der Behandlung von Reizdarmsyndrom (RDS), vorzugsweise mit einer therapeutischen Dosis von Geraniol gleich wie oder größer als 50 mg/Tag.

## Revendications

1. Composition comprenant au moins un composant d'une huile essentielle qui comprend au moins du géraniol en tant qu'ingrédient actif, la composition comprenant au moins un extrait sec de racine de gingembre supérieur ou égal à 9 % en poids sec, le composant étant adsorbé dans l'extrait sec qui définit un vecteur pour le composant, la composition résultant en une poudre huileuse qui libère l'ingrédient actif dans le gros intestin pour une utilisation dans le traitement du syndrome de l'intestin irritable (SII), de préférence à une dose thérapeutique de géraniol supérieure ou égale à 50 mg/jour.
